(19) **Europäisches Patentamt**
**European Patent Office**
**Office européen des brevets**

(11) **EP 4 130 032 A1**

(12) **EUROPEAN PATENT APPLICATION**
published in accordance with Art. 153(4) EPC

(43) Date of publication:
**08.02.2023 Bulletin 2023/06**

(21) Application number: **21774329.3**

(22) Date of filing: **25.03.2021**

(51) International Patent Classification (IPC):
*C07K 16/00* (1995.01)     *C07K 16/46* (1995.01)
*C12N 15/13* (1990.01)     *C12N 15/62* (1990.01)
*G01N 33/531* (1985.01)     *G01N 33/545* (1985.01)
*G01N 33/547* (1985.01)

(52) Cooperative Patent Classification (CPC):
**C07K 16/00; C07K 16/46; C12N 15/62;**
**G01N 33/531; G01N 33/545; G01N 33/547**

(86) International application number:
**PCT/JP2021/012724**

(87) International publication number:
**WO 2021/193870 (30.09.2021 Gazette 2021/39)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **26.03.2020 JP 2020056081**

(71) Applicant: **Denka Company Limited**
**Tokyo 103-8338 (JP)**

(72) Inventors:
• **IZUTANI, Noriyuki**
  **Tokyo 103-8338 (JP)**
• **OGASAWARA, Daisuke**
  **Tokyo 103-8338 (JP)**
• **OGURA, Hiroaki**
  **Tokyo 103-8338 (JP)**

• **MAMBA, Masako**
  **Tokyo 103-8338 (JP)**
• **NISHIMURA, Jun**
  **Tokyo 103-8338 (JP)**
• **YAMAMOTO, Kimitaka**
  **Tokyo 103-8338 (JP)**
• **ISHIKAWA, Haruto**
  **Tokyo 103-8338 (JP)**

(74) Representative: **MacLean, Martin Robert**
**Mathys & Squire**
**The Shard**
**32 London Bridge Street**
**London SE1 9SG (GB)**

Remarks:
The complete document including Reference
Table(s) and the Sequence Listing(s) can be
downloaded from the EPO website

(54) **REAGENT CONTAINING ANTIBODY HAVING PARTIAL DELETION OF FC REGION**

(57)     The present invention provides a reagent comprising an antibody from which part of CH3 or CH2 in the Fc region is deleted.

**EP 4 130 032 A1**

**Description**

**Technical Field**

The present invention generally relates to, for example, a reagent comprising an antibody from which part of CH3 or CH2 in the Fc region is deleted.

**Background Art**

Antigen-antibody reactions that use antibodies to detect and assay target substances in samples are used in the field of diagnosis by immunological methods. Immune reactions using antigen-antibody reactions may be affected by a variety of substances other than the target substance in the sample, resulting in non-specific reactions. When non-specific reactions occur, there is a risk of false positive and false negative diagnostic results.

Conventionally, a method of reducing non-specific reactions has been to remove the entire Fc region of the antibody (Patent Document 1). However, the problem has been that when an antibody fragment such as F(ab) or F(ab')$_2$ is bound to a carrier such as a microplate or latex, efficiency of binding to the carrier and activity of binding to the antigen may decline in comparison with IgG.

Citation List

Patent Document

Patent Document 1: Patent Publication JP-A-S54-119292

**Summary**

Technical Problem

Under these circumstances, it is an object of the present invention to provide, for example, a reagent comprising an antibody capable of reducing non-specific reactions.

Solution to Problem

The inventors completed the present invention by discovering that non-specific reactions in antigen-antibody reactions could be reduced simply by deleting part of the Fc region in the antibody.

That is, the present application encompasses the following inventions.

[1] A reagent comprising an antibody from which part of CH3 or CH2 in the Fc region is deleted.

[2] The reagent according to [1], wherein all of CH3 or CH2 is deleted.

[3] The reagent according to [1], wherein all of CH2 is deleted and CH3 is bound directly or indirectly to a hinge region of the antibody.

[4] The reagent according to any of [1] to [3], wherein the antibody is IgG.

[5] The region according to any of [1] to [4], wherein a non-specific reaction of the antibody is suppressed due to the deletion of part of CH3 or CH2.

[6] The reagent according to [5], wherein the non-specific reaction is caused by a non-specific reaction factor that binds to the Fc region.

[7] The reagent according to [6], wherein the non-specific reaction factor is a complement C1q, Fcγ receptor or rheumatoid factor in a sample.

[8] The reagent according to any of [1] to [7], wherein the antigen-antibody reaction between the antibody and an antigen as a target for diagnosis is at least equivalent to that between the antigen and an antibody from which the Fc region is not deleted.

[9] The reagent according to any of [1] to [8], wherein the antibody is derived from a mouse, rabbit, rat, guinea pig, hamster, goat, sheep or camel.

[10] The reagent according to any of [1] to [9], wherein the antibody is a human antibody, humanized antibody or human chimera antibody.

[11] The reagent according to any of [1] to [9], wherein the antibody is a non-human chimera antibody.

[12] The reagent according to any of [1] to [11], wherein the reagent is for diagnosis.

[13] The reagent according to any of [1] to [11], wherein the reagent is for research.

[14] A kit comprising the reagent according to any of [1] to [11].

[15] A kit comprising the reagent according to any of [1] to [11] and a blocking agent.

[16] The kit according to [14] or [15], wherein the kit comprises a carrier.

[17] The kit according to [16], wherein the antibody is bound to a surface of the carrier.

[18] The kit according to [16] or [17], wherein the carrier is a microplate or latex.

[19] The kit according to any of [16] to [18], wherein the antibody is fixed on the carrier via a linker.

[20] A method for suppressing non-specific reactions in antigen-antibody reactions, the method using an antibody from which part of CH3 or CH2 in the Fc region is deleted.

Advantageous Effects of Invention

In accordance with the present invention, a reagent capable of suppressing non-specific reactions can be provided simply by deleting part of the Fc region in an antibody. Moreover, an antibody from which part of the Fc region is deleted could improve binding efficiency to a carrier and binding activity to an antigen in comparison with F(ab')2.

**Brief Description of Drawings**

Fig. 1 shows schematic views of each ΔCH3 used in the Examples.

Fig. 2 shows schematic views of each ΔCH2 used in the Examples.

Fig. 3 shows binding results for ΔCH3 antibodies to FcγRI.

Fig. 4 shows binding results for ΔCH2 antibodies to FcγRI.

Fig. 5 shows binding results for ΔCH3 antibodies to FcγRII.

Fig. 6 shows binding results for ΔCH2 antibodies to FcγRII.

Fig. 7 shows binding results for ΔCH3 antibodies to C1q.

Fig. 8 shows binding results for ΔCH2 antibodies to C1q.

Fig. 9 shows binding results for full-length antibodies to Rf.

Fig. 10 shows binding results for ΔCH3 antibodies to Rf.

Fig. 11 shows binding results for ΔCH2 antibodies to Rf.

Fig. 12 shows the results of measurement of IgE concentrations in human samples. The black circles show measurement results using full-length antibodies, and the white circles show measurement results using CH3-deleted antibodies.

**Description of Embodiments**

(Reagent)

In the first embodiment, the present invention provides a reagent comprising an antibody from which part of CH3 or CH2 in the Fc region is deleted.

**The** antibody has a structure comprising a total of 4 polypeptide chains, two identical heavy chains and two identical light chains, bound by disulfide bonds and arranged in a Y shape. As used herein, the term "antibody" means either a natural antibody or an artificial immunoglobulin or immunoglobulin fragment that has been manufactured by complete or partial synthesis. The antibody may be an IgG, IgM, IgA, IgE or IgD isotype. Nine classes (isotypes) of human immunoglobulin are known: IgG1, IgG2, IgG3, IgG4, IgA1, IgA2, IgD, IgE and IgM. It is preferred to modify an IgG antibody. IgG antibodies as used herein is broad term, and include heavy chain antibodies (HCAb) consisting only of heavy chains, which are found in animals of the camel family such as llamas and alpacas.

The source of the antibody is not limited, and may be any mammal such as a human or a non-human animal such as a mouse, rabbit, rat, guinea pig, hamster, goat, sheep, camel or the like. In addition to human antibodies, humanized antibodies and recombinant antibodies such as chimera antibodies may also be used. The antibody may be a polyclonal antibody or a monoclonals antibody.

Antibodies are divided broadly into Fab regions associated with antigen binding and Fc regions associated with non-specific reactions and the like. When an IgG is digested with papain, it yields two Fab fragments and one Fc fragment. The Fc fragment is further divided into part of a hinge region and a CH2 region (sometimes called simply "CH2") and a CH3 region (sometimes called simply "CH3") in order from the part closest to the hinge region.

**As** used herein, "part of CH3 or CH2 in the Fc region" means at least part of either the CH2 region or the CH3 region. An antibody from which part of CH3 or CH2 in the Fc region is deleted (herein generally called an "Fc-deleted antibody", or a "CH2-deleted antibody" or "CH3-deleted antibody" depending on the deleted region) may not lack all of both the CH2 region and the CH3 region. Thus, the Fc-deleted antibody does not include F(ab) or F(ab')$_2$ fragments.

A person skilled in the art can manufacture the desired Fc-deleted antibody by known methods, such as by genetic recombination or denaturing by physical or chemical methods. For example, as described in Japanese Patent No. 5015012, the genes of the desired antibody can be expressed in a plant body, plant tissue or plant cells. Such an antibody with the deletion may also be manufactured directly, or may be manufactured from a full-length antibody that has been manufactured in advance. For example, the full-length antibody may be dissolved in a buffer, the pH of the buffer may be shifted between acidic and alkaline or heat treated to chemically and physically denature part of the Fc region, and this may then be purified by passing it through a column or the like to obtain an Fc-deleted antibody.

It is preferred that substantially all of either the CH2 or CH3 region is deleted. When the CH2 region is deleted, the CH3 then binds to the hinge region either directly or indirectly via a linker or the like. Linkers that are publicly known may be used. For example, an amino acid sequence of about 5 to 20 residues may be interposed between the hinge region and CH3. An example of such an amino acid sequence is a peptide composed of 4 continuous glycines and one serine (GS linker). The same linker may also be repeated. For example, a linker to which several (such as 2 to 5, or preferably 4) GS linkers are connected may be disposed between the hinge region and CH3 to increase the expression efficiency of the antibody.

**As** long as the desired effects are obtained, one or more (such as 1 to 20, or preferably a few, such as 1 to 10, or 1 to 5) amino acids may be deleted or substituted in the amino acid sequence of the antibody, or 1 or more (such as 1 to 20, or preferably a few, such as 1 to 10, or 1 to 5) other amino acids may be added. For example, one or more amino acids may be inserted into a cleavage region (the C-terminal end of CH2 when CH3 is deleted, or between the hinge region and CH3 when CH2 is deleted), or one or more amino acids may be deleted from the cleavage region. There are no particular limitations on the types of amino acids added, and an amino acid located at the N-terminal end of a site to be deleted may be selected. Preferably deletions and other mutations are performed in such a way as to retain the three-dimensional structure of the antibody.

The Fc-deleted antibody may also be modified with a label or the like necessary for antibody detection. Examples of labels include fluorescent substances, fluorescent proteins, enzymes, and labels with biotin or streptavidin. Other functionally necessary modifications may also be made to the Fc-deleted antibody.

**As** used herein, the term "reagent" generally means a reagent used in an immunological measurement method to detect or assay a biological substance (hereunder also called a "substance to be measured") that binds to the Fc-deleted antibody, including testing drugs and diagnostic drugs such as in-vitro diagnostic drugs. The reagent may also comprise a buffer or the like to stably maintain the antibody. Other components may be determined appropriately by a person skilled in the art according to the immunological measurement method.

Immunological measurement methods include immune staining methods (such as fluorescent antibody methods, enzyme antibody methods, heavy metal labeled antibody methods and radioactive isotope labeled antibody methods), methods combining separation by electrophoresis with detection methods using fluorescence, enzymes, radioactive isotopes and the like (such as Western blotting and fluorescent two-dimensional electrophoresis), enzyme linked immunosorbent assay (ELISA), dot blotting methods, latex agglutination-turbidimetric immunoassay (LA), immunochromatography and the like.

The substance to be measured that is detected and assayed by the reagent is not particularly limited as long as it binds to the Fc-deleted antibody, and examples include antigens such as proteins and polysaccharides, and haptens including low-molecular weight physiologically active substances such as monosaccharides, oligosaccharides, amino acids, peptides and substances with a steroid framework and the like, as well as bacteria, viruses, parasites and other pathogens composed of these. When the substance to be measured is an antigen for example, examples include, but are not limited to, protein markers such as CRP (C-reactive protein), prostate specific antigen, ferritin, $\beta$-2 microglobulin, myoglobin, hemoglobin, albumin and creatinine, immunoglobulins such as IgG, IgA and IgM, various tumor markers, lipoproteins such as LDL, HDL and TG, viral antigen such as type A influenza virus, type B influenza virus, RS virus (RSV), rhinovirus, rotavirus, norovirus, adenovirus, astrovirus, HAV, HBs, HCV, HIV and EBV, bacterial antigens such as Chlamydia trachomatis, Streptococcus, Bordetella pertussis, Helicobacter pylori, Leptospira, Treponema pallidum, Toxoplasma gondii, Borrelia, Legionella spp., anthrax and MRSA, toxins produced by bacteria and the like, microplasma lipid antigen, peptide hormones such as human chorionic gonadotropin, steroids such as steroid hormones, bioactive amines such as epinephrine and morphine, vitamins such as vitamin B, prostaglandins, antibiotics such as tetracycline, pesticides, and environmental hormones and the like. Preferred examples include antigens such as CRP, prostate-specific antigen, ferritin, $\beta$-2 microglobulin and hemoglobin.

When the substance to be measured is an antibody, examples include antibodies that interact specifically with antigens such as the aforementioned protein markers, tumor markers, lipoproteins, viral antigens, bacterial antigens, toxins produced by bacteria and the like, peptide hormones, steroids, bioactive amines, vitamins, antibiotics, pesticides, environmental hormones and the like.

The reagent is added to a sample from a subject in whom the presence or absence or the like of the substance to be measured needs to be confirmed. The sample is not particularly limited as long as it comprises the substance to be measured, and may be a bodily fluid such as blood, serum, plasma, urine, feces, sputum, interstitial fluid, spinal fluid, swab fluid or the like or a dilution of these, and blood, serum, plasma, urine, feces or spinal fluid or a dilution of these is preferred.

(Kit)

A kit comprising the reagent of the present invention is provided in a second embodiment.

The kit can be used for diagnosis, research and the like. In addition to the antibody as a reagent, the kit may also comprise a carrier for fixing the Fc-deleted antibody, and may also comprise a secondary antibody depending on the purpose of use. An insoluble carrier is preferred as such a carrier, and examples include particles such as latex particles of polyethylene, polystyrene or the like, alumina particles, silica particles, metal colloids, magnetic particles and the like. More specifically, a microplate can be used as the insoluble carrier in ELISA methods (direct method, indirect method, sandwich method, etc.), while latex or the like may be used in latex agglutination methods. To prevent non-specific reactions between the antibody and antigens, it is preferred to include a blocking agent in the kit.

The Fc-deleted antibody may be directly fixed on the carrier by physical adsorption, or may be fixed indirectly by chemical crosslinking, such as via a linker for example.

A kit used in a direct method comprises for example an antigen-binding enzyme-labeled antibody as the reagent comprising the Fc-deleted antibody, together with a substrate for the enzyme reaction, and a standard antigen or the like for use in calibration curve preparation or control experiments.

A kit used in an indirect method comprises for example an antigen-binding primary antibody as the reagent comprising the Fc-deleted antibody, a secondary antibody that binds to the primary antibody, a substrate for the enzyme reaction, and a standard antigen or the like for use in calibration curve preparation or control experiments. The secondary antibody may also be labeled for purposes of detection.

A kit for use in a sandwich method comprises for example an antibody bound to a solid phase as the reagent comprising the Fc-deleted antibody, together with an antibody for enzyme labeling, a substrate for use in the enzyme reaction, and a standard antigen or the like for use in calibration curve preparation or control experiments. The antibody for enzyme labeling may also be an Fc-deleted antibody.

A kit for use in a latex agglutination method comprises for example a binding detection antibody as the reagent comprising the Fc-deleted antibody, a standard antigen for use in calibration curve preparation or control experiments, and a blocking agent for preventing non-specific reactions between the antibody and antigens and the like.

(Method for suppressing non-specific reactions)

In a third embodiment, the present invention provides a method for using an Fc-deleted antibody to suppress non-specific reactions in an antigen-antibody reaction.

By deleting part of the Fc region, it is possible to suppress non-specific reactions that occur when non-specific reaction factors such as complement (C1q), Fc receptors, rheumatoid factors, heterophilic antibodies and the like bind to the Fc region, or in other words to CH2 and/or CH3. Because non-specific reactions can also occur when substances unrelated to the desired antigen-antibody reaction form antigen-antibody reaction products or cause cross-reactions, this is preferably used in combination with methods of non-specific reaction prevention known to those skilled in the art.

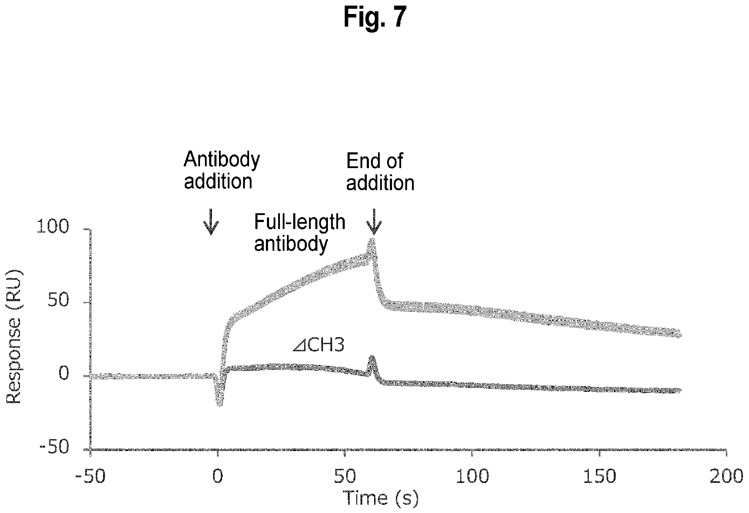

In this context, Eur J Biochem., 267(24): 7246-57 investigates the effects of the Fcγ receptor (FcγR) binding to scFv-Fc and its CH3-deleted form scFv-hCH2. From the results of Fig. 7, it appears that non-specific reactions are greater with scFv-hCH2, and therefore that it is preferable not to delete CH3 in the case of a single-stranded antibody.

The binding activity of the Fc-deleted antibody to the antigen, or in other words the antigen-antibody reaction with the antigen as the target for diagnosis, and the binding efficiency on the carrier, can be increased in comparison with an antibody from which the Fc region is not deleted.

The method for suppressing non-specific reactions includes a step of bringing the Fc-deleted antibody into contact with a sample. Any steps may also be included before and after the contact step.

The present invention is explained more concretely below with reference to examples, but the present invention is not limited by these examples.

Examples

1. Vector preparation and protein expression

An anti-H5N1 hemagglutinin antibody (Xueyong Zhu et al., "A unique and conserved neutralization epitope in H5N1 influenza viruses identified by an antibody against the A/Goose/Guangdong/1/96 hemagglutinin", Journal of virology.2013;87(23):12619-35. PMID:24049169) and an anti-butyrylcholinesterase antibody (Peng H.et al. "Comparison of 5 monoclonal antibodies for immunopurification of human butyrylcholinesterase on Dynabeads: KD values, binding pairs, and amino acid sequences", Chemico-Biological Interactions.2015;240: 336-345. DOI: 10.1016/j.cbi.2015.08.024;PMID:26343001) were selected as antibodies for making Fc-deleted antibodies.

Anti-H5N1 hemagglutinin antibody

Light chain

DIVLTQSPGSLTVSLGQRATISCRASESVDNFGKSFMHWYQQKPGQSPKLLI

YRASNREFGIPARFNGSGSGTDFALTINPVEADDVATYFCQQSNEDPRTFGGGTKL

EIKRADAAPTVSIFPPSSEQLTSGGASVVCFLNNFYPKDINVKWKIDGSERQNGVLN

SWTDQDSKDSTYSMSSTLTLTKDEYERHNSYTCEATHKTSTSPIVKSFNRNEC

(SEQ ID NO 1: GenBank KF499999)

Heavy chain

EVHLQQSGPELVKPGASVKMSCKTSGYTFTEYTIHWMKQSHGKSLEWIGGI
FPNNGDTTYNQKFKVRATLTVGRSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIE
KTISKTKGRPKAPQVYTIPPPKEQMAKDKVSLTCMITDFFPEDITVEWQWNGQPAE
NYKNTQPIMDTDGSYFVYSKLNVQKSNWEAGNTFTCSVLHEGLHNHHTEKSLSHS
PGK (SEQ ID NO 2: GenBank KF500000)

Anti-butyrylcholinesterase antibody

Light chain

DILLTQSPAILSVSPGERVRLSCRASQSCGTSIYWYQQRTNGSPRLLIMYAS
EPFSGIPSRFSGSGSGTDFTLSINSVESEDIADYYCQQSNSWPWTFGGGTRLEIKR
ADAAPTVSIFPPSSEQLTSGGASVVCFLNNFYPKDINVKWKIDGSERQNGVLNSWT
DQDSKDSTYSMSSTLTLTKDEYERHNSYTCEATHKTSTSPIVKSFNRNEC (SEQ ID
NO 3: GenBank KT189148)

Heavy chain

QVQLQQSGAELARPGASVKLSCKASGYTFTSQWLQWVKQRPGQGLEWIG
AIYPGDGDTRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD

CGCKPCICTVPEVSSVFIFPPKPKDVLTITLTPKVTCVVVDISKDDPEVQFSWFVDDV

EVHTAQTQPREEQFNSTFRSVSELPIMHQDWLNGKEFKCRVNSAAFPAPIEKTISK

TKGRPKAPQVYTIPPPKEQMAKDKVSLTCMITDFFPEDITVEWQWNGQPAENYKN

TQPIMDTDGSYFVYSKLNVQKSNWEAGNTFTCSVLHEGLHNHHTEKSLSHSPGK

(SEQ ID NO 4: GenBank KT189147)

The light chain and heavy chain of each IgG (sequence wherein its part is deleted) were each expressed in separate vectors following the methods of Example 4 of Japanese Patent No. 5015012.

1.3. ΔCH3

ΔCH3 represents antibodies from which CH3 in the heavy chain of each antibody is deleted. CH3 is the region of 341 to 447 in the EU numbering system. Consequently, the corresponding construct can be described as a protein composed of a fixed region (Fc region) consisting of the sequence of amino acids 118 to 340 in the EU numbering system. As a designation reflecting these characteristics ΔCH3 may also be called Δ341-447 or Fc 118-340. Other antibodies with such a depetion are named below according to the same rules. A GS linker (GGGGS) and an His tag (HHHHHH) are attached to the C end of ΔCH3.

1.3.1. ΔCH3 = Δ341-447 = Fc 118-340:

1.3.1.1. Anti-H5N1 hemagglutinin antibody

EVHLQQSGPELVKPGASVKMSCKTSGYTFTEYTIHWMKQSHGKSLEWIGGI

FPNNGDTTYNQKFKVRATLTVGRSSSTAYMDLRSLTSEDSAVYYCVRNYGSSYGY

FDVWGAGTTVTVSSAKTTPPSVYPLAPGSAAQTNSMVTLGCLVKGYFPEPVTVTW

NSGSLSSGVHTFPAVLQSDLYTLSSSVTVPSSTWPSETVTCNVAHPASSTKVDKKI

VPRDCGCKPCICTVPEVSSVFIFPPKPKDVLTITLTPKVTCVVVDISKDDPEVQFSWF

VDDVEVHTAQTQPREEQFNSTFRSVSELPIMHQDWLNGKEFKCRVNSAAFPAPIE

KTISKTKGGGGSHHHHHH (SEQ ID NO: 5)

1.3.1.2 Anti-butyrylcholinesterase antibody

QVQLQQSGAELARPGASVKLSCKASGYTFTSQWLQWVKQRPGQGLEWIG

AIYPGDGDTRYTQFKGKATLTADKSSSTAYMQLTNLAPEDSAVYYCARSSMDYW

GQGTSVTVSSAKTTPPSVYPLAPGSAAQTNSMVTLGCLVKGYFPEPVTVTWNSGS

LSSGVHTFPAVLQSDLYTLSSSVTVPSSTWPSETVTCNVAHPASSTKVDKKIVPRD

CGCKPCICTVPEVSSVFIFPPKPKDVLTITLTPKVTCVVVDISKDDPEVQFSWFVDDV

EVHTAQTQPREEQFNSTFRSVSELPIMHQDWLNGKEFKCRVNSAAFPAPIEKTISK

TKGGGGSHHHHHH (SEQ ID NO: 6)

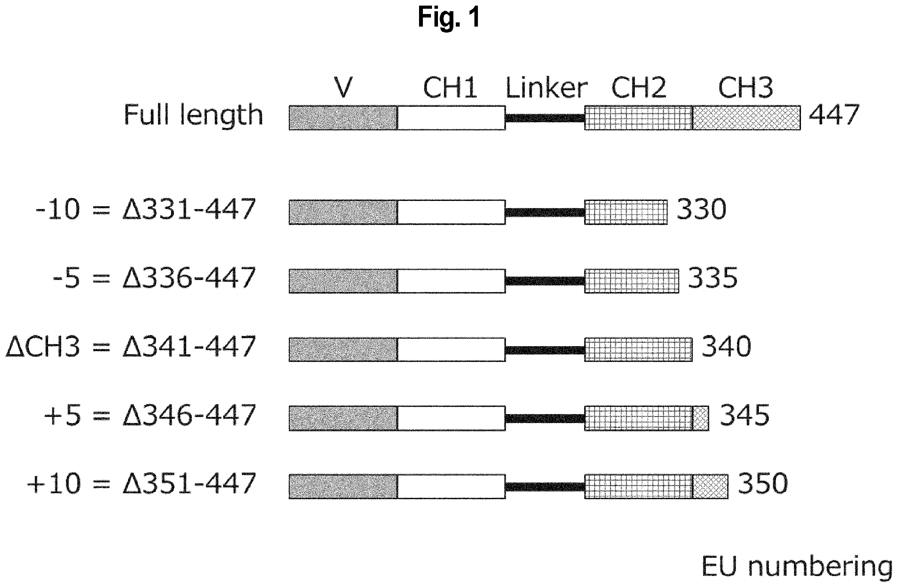

To confirm the effects of the deletion, 4 different kinds of ΔCH3 were prepared having 5 amino acids deleted or added or having 10 amino acids deleted or added beginning from the cleavage site as shown in Fig. 1. The specific sequences are given below.

1.3.2. Δ331-447 = Fc 118-330 (-10 amino acids):

1.3.2.1. Anti-H5N1 hemagglutinin antibody

EVHLQQSGPELVKPGASVKMSCKTSGYTFTEYTIHWMKQSHGKSLEWIGGI

FPNNGDTTYNQKFKVRATLTVGRSSSTAYMDLRSLTSEDSAVYYCVRNYGSSYGY

FDVWGAGTTVTVSSAKTTPPSVYPLAPGSAAQTNSMVTLGCLVKGYFPEPVTVTW

NSGSLSSGVHTFPAVLQSDLYTLSSSVTVPSSTWPSETVTCNVAHPASSTKVDKKI

VPRDCGCKPCICTVPEVSSVFIFPPKPKDVLTITLTPKVTCVVVDISKDDPEVQFSWF

VDDVEVHTAQTQPREEQFNSTFRSVSELPIMHQDWLNGKEFKCRVNSAAFPAGG

GGSHHHHHH (SEQ ID NO: 7)

1.3.2.2. Anti-butyrylcholinesterase antibody

QVQLQQSGAELARPGASVKLSCKASGYTFTSQWLQWVKQRPGQGLEWIG
AIYPGDGDTRYTQFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTVPEVSSVFIFPPKPKDVLTITLTPKVTCVVVDISKDDPEVQFSWFVDDV
EVHTAQTQPREEQFNSTFRSVSELPIMHQDWLNGKEFKCRVNSAAFPAGGGGSH
HHHHH (SEQ ID NO: 8)

1.3.3. Δ336-447 = Fc 118-335 (-5 amino acids):

1.3.3.1. Anti-H5N1 hemagglutinin antibody

EVHLQQSGPELVKPGASVKMSCKTSGYTFTEYTIHWMKQSHGKSLEWIGGI
FPNNGDTTYNQKFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIE
KTGGGGSHHHHHH (SEQ ID NO: 9)

1.3.3.2. Anti-butyrylcholinesterase antibody

QVQLQQSGAELARPGASVKLSCKASGYTFTSQWLQWVKQRPGQGLEWIG
AIYPGDGDTRYTQFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTVPEVSSVFIFPPKPKDVLTITLTPKVTCVVVDISKDDPEVQFSWFVDDV
EVHTAQTQPREEQFNSTFRSVSELPIMHQDWLNGKEFKCRVNSAAFPAPIEKTGG
GGSHHHHHH (SEQ ID NO: 10)

1.3.4. Δ346-447 = Fc 118-345 (+5 amino acids)

1.3.4.1. Anti-H5N1-hemagglutinin antibody

EVHLQQSGPELVKPGASVKMSCKTSGYTFTEYTIHWMKQSHGKSLEWIGGI
FPNNGDTTYNQKFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIE
KTISKTKGRPKAGGGGSHHHHHH (SEQ ID NO: 11)

1.3.4.2. Anti-butyrylcholinesterase antibody

QVQLQQSGAELARPGASVKLSCKASGYTFTSQWLQWVKQRPGQGLEWIG AIYPGDGDTRYTQFKGKATLTADKSSSTAYMQLTNLAPEDSAVYYCARSSMDYW GQGTSVTVSSAKTTPPSVYPLAPGSAAQTNSMVTLGCLVKGYFPEPVTVTWNSGS LSSGVHTFPAVLQSDLYTLSSSVTVPSSTWPSETVTCNVAHPASSTKVDKKIVPRD CGCKPCICTVPEVSSVFIFPPKPKDVLTITLTPKVTCVVVDISKDDPEVQFSWFVDDV EVHTAQTQPREEQFNSTFRSVSELPIMHQDWLNGKEFKCRVNSAAFPAPIEKTISK TKGRPKAGGGGSHHHHHH (SEQ ID NO: 12)

1.3.5. Δ351-447 = Fc 118-350 (+10 amino acids):

1.3.5.1. Anti-H5N1 hemagglutinin antibody

EVHLQQSGPELVKPGASVKMSCKTSGYTFTEYTIHWMKQSHGKSLEWIGGI FPNNGDTTYNQKFKVRATLTVGRSSSTAYMDLRSLTSEDSAVYYCVRNYGSSYGY FDVWGAGTTVTVSSAKTTPPSVYPLAPGSAAQTNSMVTLGCLVKGYFPEPVTVTW NSGSLSSGVHTFPAVLQSDLYTLSSSVTVPSSTWPSETVTCNVAHPASSTKVDKKI VPRDCGCKPCICTVPEVSSVFIFPPKPKDVLTITLTPKVTCVVVDISKDDPEVQFSWF VDDVEVHTAQTQPREEQFNSTFRSVSELPIMHQDWLNGKEFKCRVNSAAFPAPIE KTISKTKGRPKAPQVYTGGGGSHHHHHH (SEQ ID NO: 13)

1.3.5.2. Anti-butyrylcholinesterase antibody

QVQLQQSGAELARPGASVKLSCKASGYTFTSQWLQWVKQRPGQGLEWIG AIYPGDGDTRYTQFKGKATLTADKSSSTAYMQLTNLAPEDSAVYYCARSSMDYW GQGTSVTVSSAKTTPPSVYPLAPGSAAQTNSMVTLGCLVKGYFPEPVTVTWNSGS LSSGVHTFPAVLQSDLYTLSSSVTVPSSTWPSETVTCNVAHPASSTKVDKKIVPRD CGCKPCICTVPEVSSVFIFPPKPKDVLTITLTPKVTCVVVDISKDDPEVQFSWFVDDV EVHTAQTQPREEQFNSTF (SEQ ID NO: 14)

1.4. ΔCH2

For the ΔCH2, the CH2 of the heavy chain was deleted from an anti-IgE antibody, and the hinge regions on either side were linked to CH3. CH2 is the region from 231 to 340 in the EU numbering system. Consequently, the corresponding construct can be described as a protein composed of a fixed region (Fc region) consisting of the sequence of amino acids 118 to 230 linked to amino acids 341 to 447 in the EU numbering system.

Considering the possible effects of CH2 deletion, antibodies were prepared with GS linkers (GGGGS) inserted into the connecting parts. The GS linker was repeated 1 to 4 times. The specific amino acid sequences were 1) without linker, 2) with GGGGS (SEQ ID NO: 15), 3) with GGGGSGGGGS (SEQ ID NO: 16), 4) with GGGGSGGGGSGGGGS (SEQ ID NO: 17) and 5) with GGGGSGGGGSGGGGSGGGGS (SEQ ID NO: 18). The sequence 5) with 4 repetitions of the GS linker was used to obtain data for evaluating non-specific reactions. The specific sequences are given below.

1.4.1. ΔCH2 = Δ231-340 = Fc 118-230 + 341-447 (no linker)

1.4.1.1. Anti-H5N1 hemagglutinin antibody

EVHLQQSGPELVKPGASVKMSCKTSGYTFTEYTIHWMKQSHGKSLEWIGGI

FPNNGDTTYNQKFKVRATLTVGRSSSTAYMDLRSLTSEDSAVYYCVRNYGSSYGY

FDVWGAGTTVTVSSAKTTPPSVYPLAPGSAAQTNSMVTLGCLVKGYFPEPVTVTW

NSGSLSSGVHTFPAVLQSDLYTLSSSVTVPSSTWPSETVTCNVAHPASSTKVDKKI

VPRDCGCKPCICTGRPKAPQVYTIPPPKEQMAKDKVSLTCMITDFFPEDITVEWQW

NGQPAENYKNTQPIMDTDGSYFVYSKLNVQKSNWEAGNTFTCSVLHEGLHNHHTE

KSLSHSPGKGGGGSHHHHHH (SEQ ID NO: 19)

1.4.1.2. Anti-butyrylcholinesterase antibody

QVQLQQSGAELARPGASVKLSCKASGYTFTSQWLQWVKQRPGQGLEWIG

AIYPGDGDTRYTQKFKGKATLTADKSSSTAYMQLTNLAPEDSAVYYCARSSMDYW

GQGTSVTVSSAKTTPPSVYPLAPGSAAQTNSMVTLGCLVKGYFPEPVTVTWNSGS

LSSGVHTFPAVLQSDLYTLSSSVTVPSSTWPSETVTCNVAHPASSTKVDKKIVPRD

CGCKPCICTGRPKAPQVYTIPPPKEQMAKDKVSLTCMITDFFPEDITVEWQWNGQP

AENYKNTQPIMDTDGSYFVYSKLNVQKSNWEAGNTFTCSVLHEGLHNHHTEKSLS

HSPGKGGGGSHHHHHH (SEQ ID NO: 20)

1.4.2. ΔCH2 = Δ231-340 = Fc 118-230 + 341-447 (1 × GS linker):

1.4.2.1. Anti-H5N1 hemagglutinin antibody

EVHLQQSGPELVKPGASVKMSCKTSGYTFTEYTIHWMKQSHGKSLEWIGGI
FPNNGDTTYNQKFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTGGGGSGRPKAPQVYTIPPPKEQMAKDKVSLTCMITDFFPEDIT
VEWQWNGQPAENYKNTQPIMDTDGSYFVYSKLNVQKSNWEAGNTFTCSVLHEGL
HNHHTEKSLSHSPGKGGGGSHHHHHH (SEQ ID NO: 21)

1.4.2.2. Anti-butyrylcholinesterase antibody

QVQLQQSGAELARPGASVKLSCKASGYTFTSQWLQWVKQRPGQGLEWIG
AIYPGDGDTRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTGGGGSGRPKAPQVYTIPPPKEQMAKDKVSLTCMITDFFPEDITVEWQ
WNGQPAENYKNTQPIMDTDGSYFVYSKLNVQKSNWEAGNTFTCSVLHEGLHNHH
TEKSLSHSPGKGGGGSHHHHHH (SEQ ID NO: 22)

1.4.3. ΔCH2 = Δ231-340 = Fc 118-230 + 341-447 (2 × GS linker):

1.4.3.1. Anti-H5N1 hemagglutinin antibody

EVHLQQSGPELVKPGASVKMSCKTSGYTFTEYTIHWMKQSHGKSLEWIGGI
FPNNGDTTYNQFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTGGGGSGGGGSGRPKAPQVYTIPPPKEQMAKDKVSLTCMITDF
FPEDITVEWQWNGQPAENYKNTQPIMDTDGSYFVYSKLNVQKSNWEAGNTFTCS
VLHEGLHNHHTEKSLSHSPGKGGGGSHHHHHH (SEQ ID NO: 23)

1.4.3.2. Anti-butyrylcholinesterase antibody

QVQLQQSGAELARPGASVKLSCKASGYTFTSQWLQWVKQRPGQGLEWIG
AIYPGDGDTRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTGGGGSGGGGSGRPKAPQVYTIPPPKEQMAKDKVSLTCMITDFFPEDI
TVEWQWNGQPAENYKNTQPIMDTDGSYFVYSKLNVQKSNWEAGNTFTCSVLHEG
LHNHHTEKSLSHSPGKGGGGSHHHHHH (SEQ ID NO: 24)

1.4.4. ΔCH2 = Δ231-340 = Fc 118-230 + 341-447 (3 × GS linker):

1.4.4.1. Anti-H5N1 hemagglutinin antibody

EVHLQQSGPELVKPGASVKMSCKTSGYTFTEYTIHWMKQSHGKSLEWIGGI FPNNGDTTYNQKFKVRATLTVGRSSSTAYMDLRSLTSEDSAVYYCVRNYGSSYGY FDVWGAGTTVTVSSAKTTPPSVYPLAPGSAAQTNSMVTLGCLVKGYFPEPVTVTW NSGSLSSGVHTFPAVLQSDLYTLSSSVTVPSSTWPSETVTCNVAHPASSTKVDKKI VPRDCGCKPCICTGGGGSGGGGSGGGGSGRPKAPQVYTIPPPKEQMAKDKVSLT CMITDFFPEDITVEWQWNGQPAENYKNTQPIMDTDGSYFVYSKLNVQKSNWEAGN TFTCSVLHEGLHNHHTEKSLSHSPGKGGGGSHHHHHH (SEQ ID NO: 25)

1.4.4.2. Anti-butyrylcholinesterase antibody

QVQLQQSGAELARPGASVKLSCKASGYTFTSQWLQWVKQRPGQGLEWIG AIYPGDGDTRYTQKFKGKATLTADKSSSTAYMQLTNLAPEDSAVYYCARSSMDYW GQGTSVTVSSAKTTPPSVYPLAPGSAAQTNSMVTLGCLVKGYFPEPVTVTWNSGS LSSGVHTFPAVLQSDLYTLSSSVTVPSSTWPSETVTCNVAHPASSTKVDKKIVPRD CGCKPCICTGGGGSGGGGSGGGGSGRPKAPQVYTIPPPKEQMAKDKVSLTCMIT DFFPEDITVEWQWNGQPAENYKNTQPIMDTDGSYFVYSKLNVQKSNWEAGNTFT CSVLHEGLHNHHTEKSLSHSPGKGGGGSHHHHHH (SEQ ID NO: 26)

1.4.5. ΔCH2 = Δ231-340 = Fc 118-230 + 341-447 (4 × GS linker):

1.4.5.1. Anti-H5N1 hemagglutinin antibody

EVHLQQSGPELVKPGASVKMSCKTSGYTFTEYTIHWMKQSHGKSLEWIGGI FPNNGDTTYNQKFKVRATLTVGRSSSTAYMDLRSLTSEDSAVYYCVRNYGSSYGY

FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTGGGGSGGGGSGGGGSGGGGSGRPKAPQVYTIPPPKEQMAK
DKVSLTCMITDFFPEDITVEWQWNGQPAENYKNTQPIMDTDGSYFVYSKLNVQKS
NWEAGNTFTCSVLHEGLHNHHTEKSLSHSPGKGGGGSHHHHHH (SEQ ID NO: 27)

1.4.5.2. Anti-butyrylcholinesterase antibody

QVQLQQSGAELARPGASVKLSCKASGYTFTSQWLQWVKQRPGQGLEWIG
AIYPGDGDTRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTGGGGSGGGGSGGGGSGGGGSGRPKAPQVYTIPPPKEQMAKDKVS
LTCMITDFFPEDITVEWQWNGQPAENYKNTQPIMDTDGSYFVYSKLNVQKSNWEA
GNTFTCSVLHEGLHNHHTEKSLSHSPGKGGGGSHHHHHH (SEQ ID NO: 28)

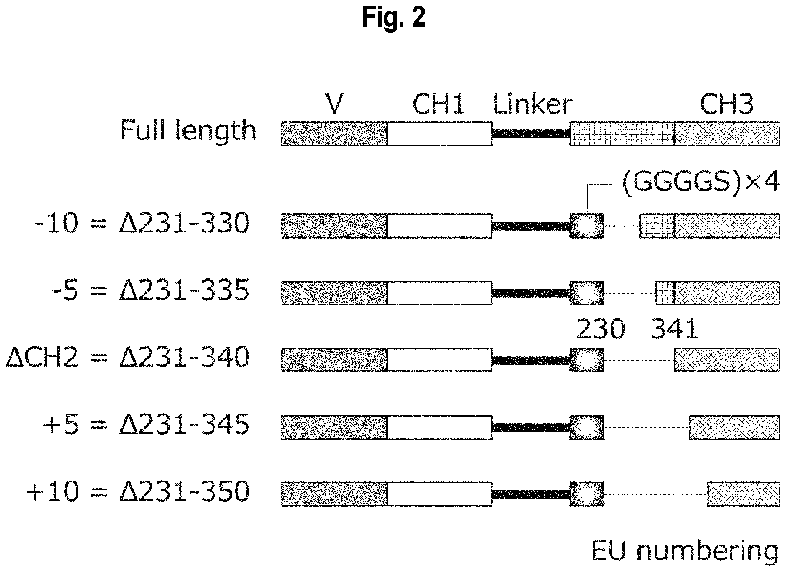

To confirm the effects of the deletions, 4 different kinds of ΔCH2 were prepared having 5 amino acids deleted or added or having 10 amino acids deleted or added beginning from the C-terminal cleavage site as shown in Fig. 2. The specific sequences are given below.

1.4.6. ΔCH2 = Δ231-330 = Fc 118-230 + 331-447 (4 × GS linker) (-10 amino acids):

1.4.6.1. Anti-H5N1 hemagglutinin antibody

EVHLQQSGPELVKPGASVKMSCKTSGYTFTEYTIHWMKQSHGKSLEWIGGI
FPNNGDTTYNQKFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW

NSGSLSSGVHTFPAVLQSDLYTLSSSVTVPSSTWPSETVTCNVAHPASSTKVDKKI VPRDCGCKPCICTGGGGSGGGGSGGGGSGGGGSPIEKTISKTKGRPKAPQVYTIP PPKEQMAKDKVSLTCMITDFFPEDITVEWQWNGQPAENYKNTQPIMDTDGSYFVY SKLNVQKSNWEAGNTFTCSVLHEGLHNHHTEKSLSHSPGK (SEQ ID NO: 29)

1.4.6.2. Anti-butyrylcholinesterase antibody

QVQLQQSGAELARPGASVKLSCKASGYTFTSQWLQWVKQRPGQGLEWIG AIYPGDGDTRYTQKFKGKATLTADKSSSTAYMQLTNLAPEDSAVYYCARSSMDYW GQGTSVTVSSAKTTPPSVYPLAPGSAAQTNSMVTLGCLVKGYFPEPVTVTWNSGS LSSGVHTFPAVLQSDLYTLSSSVTVPSSTWPSETVTCNVAHPASSTKVDKKIVPRD CGCKPCICTGGGGSGGGGSGGGGSGGGGSPIEKTISKTKGRPKAPQVYTIPPPKE QMAKDKVSLTCMITDFFPEDITVEWQWNGQPAENYKNTQPIMDTDGSYFVYSKLN VQKSNWEAGNTFTCSVLHEGLHNHHTEKSLSHSPGK (SEQ ID NO: 30)

1.4.7. ΔCH2 = Δ231-335 = Fc 118-230 + 336-447 (4 × GS linker) (-5 amino acids):

1.4.7.1. Anti-H5N1 hemagglutinin antibody

EVHLQQSGPELVKPGASVKMSCKTSGYTFTEYTIHWMKQSHGKSLEWIGGI FPNNGDTTYNQKFKVRATLTVGRSSSTAYMDLRSLTSEDSAVYYCVRNYGSSYGY FDVWGAGTTVTVSSAKTTPPSVYPLAPGSAAQTNSMVTLGCLVKGYFPEPVTVTW NSGSLSSGVHTFPAVLQSDLYTLSSSVTVPSSTWPSETVTCNVAHPASSTKVDKKI VPRDCGCKPCICTGGGGSGGGGSGGGGSGGGGSISKTKGRPKAPQVYTIPPPKE QMAKDKVSLTCMITDFFPEDITVEWQWNGQPAENYKNTQPIMDTDGSYFVYSKLN VQKSNWEAGNTFTCSVLHEGLHNHHTEKSLSHSPGK (SEQ ID NO: 31)

1.4.7.2. Anti-butyrylcholinesterase antibody

QVQLQQSGAELARPGASVKLSCKASGYTFTSQWLQWVKQRPGQGLEWIG
AIYPGDGDRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTGGGGSGGGGSGGGGSGGGGSISKTKGRPKAPQVYTIPPPKEQMAK
DKVSLTCMITDFFPEDITVEWQWNGQPAENYKNTQPIMDTDGSYFVYSKLNVQKS
NWEAGNTFTCSVLHEGLHNHHTEKSLSHSPGK (SEQ ID NO: 32)

1.4.8. ΔCH2 = Δ231-345 = Fc 118-230 + 346-447 (4 × GS linker) (+5 amino acids):

1.4.8.1. Anti-H5N1 hemagglutinin antibody

EVHLQQSGPELVKPGASVKMSCKTSGYTFTEYTIHWMKQSHGKSLEWIGGI
FPNNGDTTYNQKFKVRATLTVGRSSSTAYMDLRSLTSEDSAVYYCVRNYGSSYGY
FDVWGAGTTVTVSSAKTTPPSVYPLAPGSAAQTNSMVTLGCLVKGYFPEPVTVTW
NSGSLSSGVHTFPAVLQSDLYTLSSSVTVPSSTWPSETVTCNVAHPASSTKVDKKI
VPRDCGCKPCICTGGGGSGGGGSGGGGSGGGGSPQVYTIPPPKEQMAKDKVSL
TCMITDFFPEDITVEWQWNGQPAENYKNTQPIMDTDGSYFVYSKLNVQKSNWEAG
NTFTCSVLHEGLHNHHTEKSLSHSPGK (SEQ ID NO: 33)

1.4.8.2. Anti-butyrylcholinesterase antibody

QVQLQQSGAELARPGASVKLSCKASGYTFTSQWLQWVKQRPGQGLEWIG
AIYPGDGDRYTQKFKGKATLTADKSSSTAYMQLTNLAPEDSAVYYCARSSMDYW
GQGTSVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRD
CGCKPCICTGGGGSGGGGSGGGGSGGGGSPQVYTIPPPKEQMAKDKVSLTCMIT

DFFPEDITVEWQWNGQPAENYKNTQPIMDTDGSYFVYSKLNVQKSNWEAGNTFT

CSVLHEGLHNHHTEKSLSHSPGK (SEQ ID NO: 34)

1.4.9. ΔCH2 = Δ231-350 = Fc 118-230 + 351-447 (4 × GS linker) (+10 amino acids):

1.4.9.1. Anti-H5N1 hemagglutinin antibody

EVHLQQSGPELVKPGASVKMSCKTSGYTFTEYTIHWMKQSHGKSLEWIGGI

FPNNGDTTYNQKFKVRATLTVGRSSSTAYMDLRSLTSEDSAVYYCVRNYGSSYGY

FDVWGAGTTVTVSSAKTTPPSVYPLAPGSAAQTNSMVTLGCLVKGYFPEPVTVTW

NSGSLSSGVHTFPAVLQSDLYTLSSSVTVPSSTWPSETVTCNVAHPASSTKVDKKI

VPRDCGCKPCICTGGGGSGGGGSGGGGSGGGGSIPPPKEQMAKDKVSLTCMITD

FFPEDITVEWQWNGQPAENYKNTQPIMDTDGSYFVYSKLNVQKSNWEAGNTFTC

SVLHEGLHNHHTEKSLSHSPGK (SEQ ID NO: 35)

1.4.9.2. Anti-butyrylcholinesterase antibody

QVQLQQSGAELARPGASVKLSCKASGYTFTSQWLQWVKQRPGQGLEWIG

AIYPGDGDTRYTQKFKGKATLTADKSSSTAYMQLTNLAPEDSAVYYCARSSMDYW

GQGTSVTVSSAKTTPPSVYPLAPGSAAQTNSMVTLGCLVKGYFPEPVTVTWNSGS

LSSGVHTFPAVLQSDLYTLSSSVTVPSSTWPSETVTCNVAHPASSTKVDKKIVPRD

CGCKPCICTGGGGSGGGGSGGGGSGGGGSIPPPKEQMAKDKVSLTCMITDFFPE

DITVEWQWNGQPAENYKNTQPIMDTDGSYFVYSKLNVQKSNWEAGNTFTCSVLH

EGLHNHHTEKSLSHSPGK (SEQ ID NO: 36)

**The** light chain of IgG was cloned to a tobacco mosaic virus (TMV) vector (manufactured by Icon Genetics), and each heavy chain (including CH3-deleted or CH2-deleted forms) was cloned to a potato mosaic virus X (PVX) vector (Icon Genetics). Each of these vectors was separately introduced into a different agrobacterium, the resulting transformants were cultured, and a mixture of the culture liquids was infiltrated into Nicotinia benthamiana leaves, which were harvested after about 1 week. The collected leaves (infected leaves) were freeze dried.

**2.** Purification

500 g of the freeze-dried infected leaves was weighed and crushed with a cutter mixer. 250 ml of extraction solution (100 mM Tris, 250 mM NaCl, 40 mM sodium ascorbate) was added to the crushed material, and the infected leaves were repeatedly crushed.

The crushed infected leaves collected from the cutter mixer were mixed with crushed infected leaves obtained by washing the inside of the mixer with 250 ml of new extraction solution. A part of this was sampled and centrifuged for 5 minutes as 12,000 × g, and the supernatant (extraction step fraction) was collected.

The pH of the extraction step fraction was adjusted, and this was filtered with an 0.45 μm filter and then with an 0.22 μm filter. The filtrate was subjected to nickel column chromatography (GE Health Care, HisPrep FF 16/10 column, Catalog No. 28936551) and HAP column chromatography to remove plant-derived impurities and antibody-derived impurities.

3. Expression test

Three times the amount of extraction buffer (100 mM Tris, 250 mM NaCl, 5 mM EDTA) was added to the infected leaves, which were then crushed with a bead mill and left for 30 minutes on ice. This was centrifuged (8000 xg, 4°C, 10 minutes), and the supernatant was collected and analyzed by SDS-PAGE and Western blotting.

Expression of the antibodies shown in Tables 1 and 2 was evaluated by Western blotting using HRP-labeled His-tag antibodies. Expression of all antibodies was confirmed.

[Table 1]

|   | Antibody | ΔCH3 form |
|---|---|---|
| 1 | Anti-IgE antibody | ΔCH3 = Δ341-447 |
| 2 | Anti-butyrylcholinesterase antibody | +5 = Δ346-447 |
| 3 | | +10 = Δ351-447 |
| 4 | Anti-H5N1 hemagglutinin antibody | +5 = Δ346-447 |
| 5 | | +10 = Δ351-447 |

[Table 2]

|   | Antibody | ΔCH2 form |
|---|---|---|
| 1 | Anti-IgE antibody | ΔCH2 = Δ231-340 |
| 2 | Anti-butyrylcholinesterase antibody | −5 = Δ231-335 |
| 3 | | −10 = Δ231-330 |
| 4 | | +5 = Δ231-345 |
| 5 | Anti-H5N1 hemagglutinin antibody | −5 = Δ231-335 |
| 6 | | −10 = Δ231-330 |
| 7 | | +5 = Δ231-345 |

Expression of a camel ΔCH3 antibody was also confirmed.

**4.** Non-specific evaluation

Interactions between the purified antibodies and non-specific factors was evaluated by surface plasmon resonance (SPR). A BIAcore X-100 (manufactured by Biacore) was used as the equipment. Fc receptors (Fc gamma receptor I, Fc gamma receptor II), C1q and rheumatoid factor were used as the non-specific factors.

<Fc receptors>

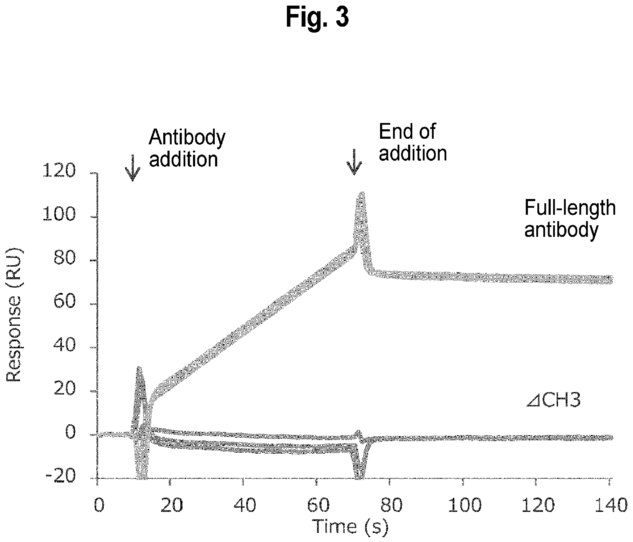

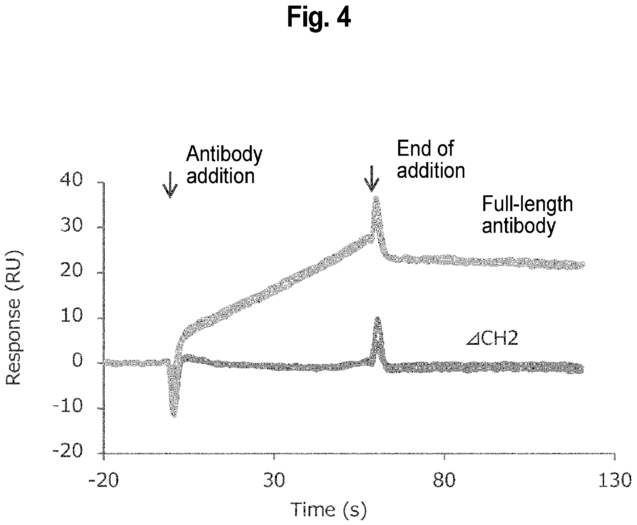

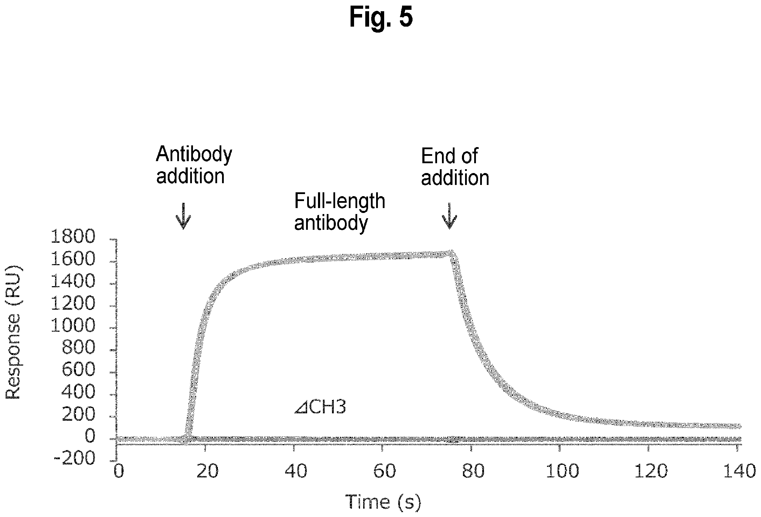

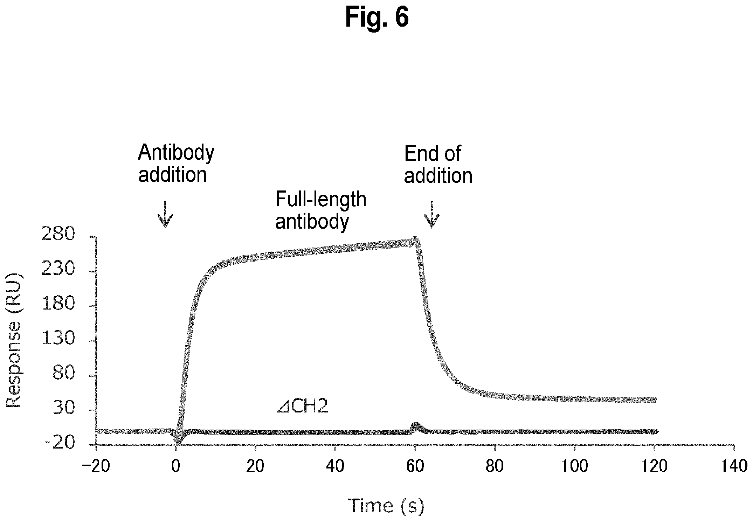

Biotin-labeled human-derived FcγRI or FcγRII (manufactured by Sino Biological, all purified) were captured on Sensor Chip CAP (GE Health Care), and increases in a signal (Resonance units: RU) associated with antibody interactions were observed. Using 0.01 M HEPES pH 7.4, 0.15 M NaCl, 3 mM EDTA, 0.05% Tween20 as the running buffer, measurement was performed at 25°C at a rate of 30 μL/minute. n = 3 measurements were performed in each case (here and below). The binding effects of the antibodies to FcγRI are shown in Fig. 3 and Fig. 4, and the binding effects of the antibodies to FcγRII are shown in Fig. 5 and Fig. 6.

When 1 μM of the antibody was added for 1 minute, interactions were observed as shown by an increase in the signal with the full-length antibody, but with ΔCH3 the signal was unchanged, indicating that no interactions had occurred (Fig. 3 and Fig. 5). With ΔCH2, similarly, interactions were observed with the full-length antibody when 0.25 μM of the antibody was added for 1 minute, but with ΔCH2 the signal was unchanged, indicating that no interactions had occurred (Fig. 4 and Fig. 6).

<C1q>

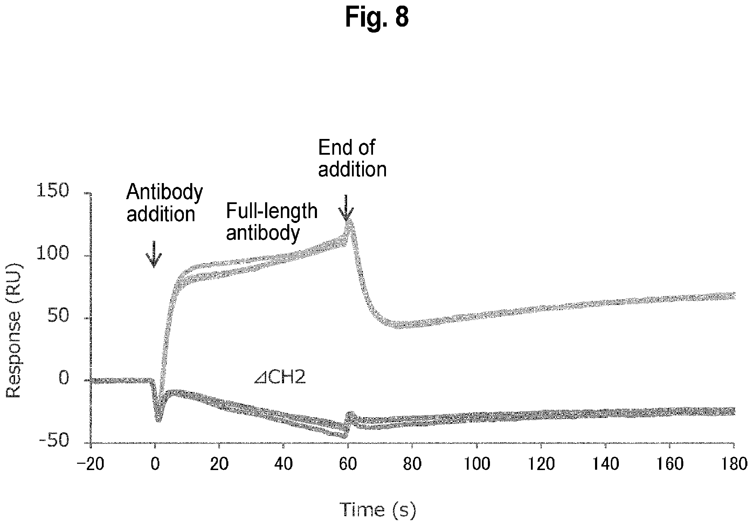

The antibodies were captured on a Sensor chip Protein L (GE Health Care), and increases in a signal (resonance units: RU) associated with interactions with human-derived C1q (Abcam Co. ab96363, purified product) were observed. Using 0.01 M HEPES pH 7.4, 0.15 M NaCl, 3 mM EDTA, 0.05% Tween20 as the running buffer, measurement was performed at 25°C at a rate of 30 μL/minute. The binding effects of the antibodies to C1q are shown in Fig. 7 and Fig. 8.

When human derived C1q diluted 100 times with running buffer was added for 1 minute, neither ΔCH3 nor ΔCH2 exhibited any interactions with C1q.

<Rf>

The antibodies were fixed to a Sensor chip CM5 (GE Health Care) based on the amine coupling method, and increases in a signal (Resonance units: RU) associated with interactions with Rf-positive human plasma or serum were observed. Using 0.01 M HEPES pH 7.4, 0.15 M NaCl, 3 mM EDTA, 0.05% Tween20 as the running buffer, measurement was performed at 25°C at a rate of 30 μL/minute.

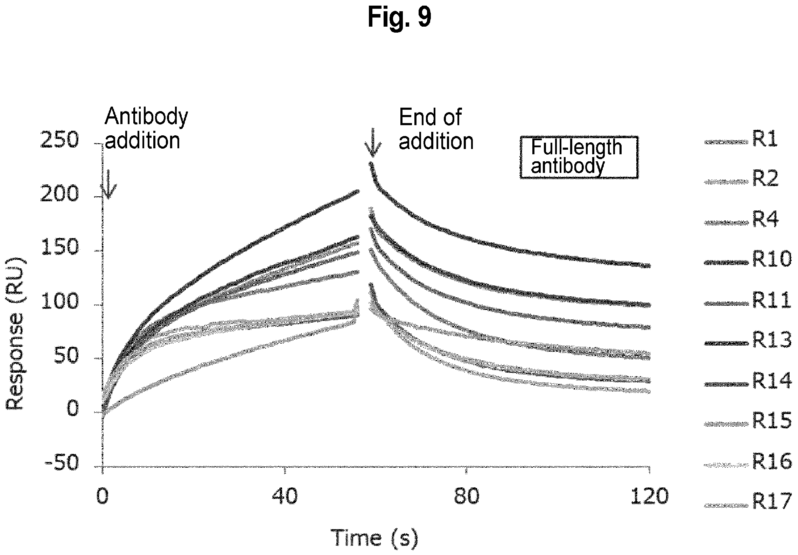

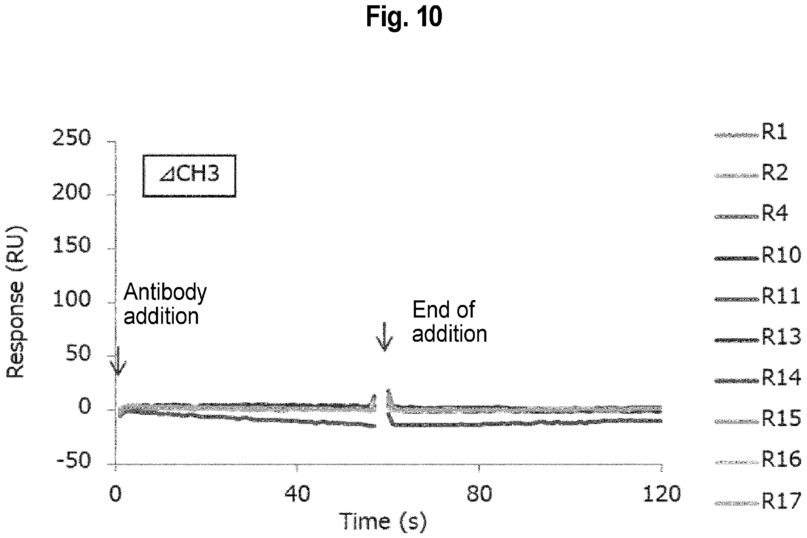

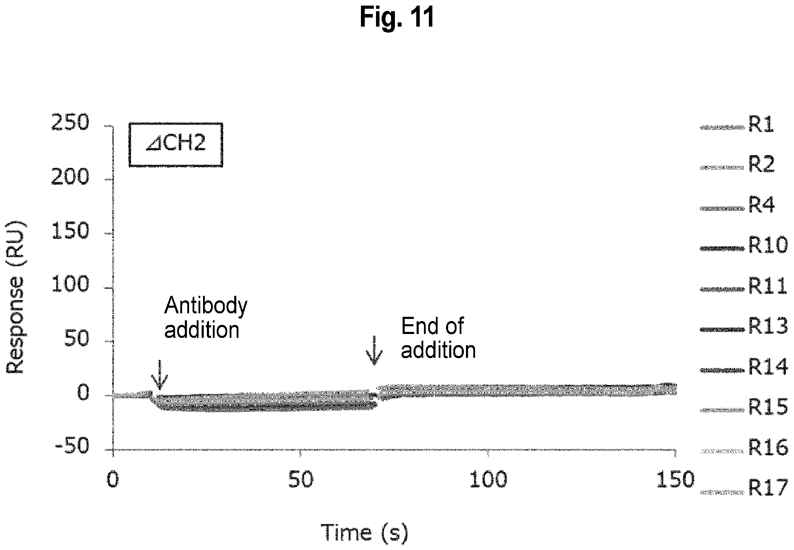

Upon evaluating 10 kinds of Rf-positive human serum or plasma that had been shown to contain factors that interact with the full-length antibody, interactions were observed as shown by an increase in the signal with the full-length antibody when serum or plasma diluted 30 times with running buffer was added for 1 minute (Fig. 9). No interactions were confirmed with either ΔCH3 or ΔCH2 (Fig. 10 and Fig. 11).

5. Latex agglutination test

Binding efficiency of the resulting antibodies to latex was evaluated by the following procedures.

(1) Preparing reagent

A measurement reagent using the immune agglutination method was prepared as follows using an IgE antibody.

i) 0.1 mg of the antibody was carried on 1 ml of a polystyrene latex suspension with an average particle diameter of 200 nm to obtain sensitized particles that were then suspended to 0.1% in buffer (glycine, pH 7.3) to prepare a latex suspension.

ii) A buffer (glycine, pH 8.3) was prepared.

**(2)** Measurement using automatic analyzer

Automatic measurement was performed by the endpoint method using a Hitachi 7180 automatic analyzer. Using the reagent prepared in i) of (1) above, measurement was performed on 4 human samples that were known to exhibit false-positives due to non-specific reactions and 15 samples that did not cause non-specific reactions. 140 μL of the buffer prepared in ii) of (1) above was added to 3.5 μL of the sample solution, and this mixture was stirred and mixed at 37°C. This was left for 5 minutes, 70 μL of the latex suspension was added, and this was further stirred and mixed at 37°C. The agglutination reaction was measured for about 5 minutes based on changes in absorbance, and the IgE concentration of each sample was calculated based on a calibration curve.

(3) Comparing measurement values for false-positive samples

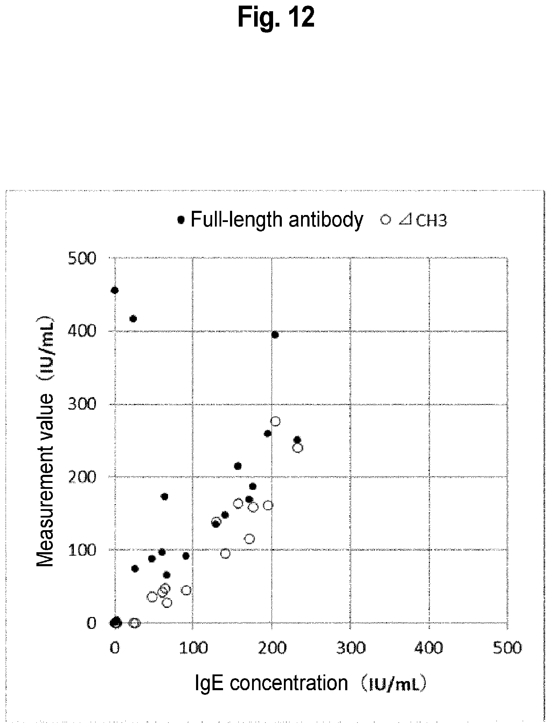

The results of measurement in (2) above are shown in Fig. 12 and Table 3.

[Table 3]

| Sample No. | IgE concentration | Measurement value | |
|---|---|---|---|
| | | Full-length antibody | △CH3 |
| No. 1 | 0 | 455 | 0 |
| No. 2 | 0 | 2 | 0 |
| No. 3 | 2 | 1 | 0 |
| No. 4 | 3 | 4 | 0 |
| No. 5 | 24 | 416 | 0 |
| No. 6 | 26 | 74 | 0 |
| No. 7 | 47 | 88 | 36 |
| No. 8 | 61 | 97 | 42 |
| No. 9 | 64 | 173 | 47 |
| No. 10 | 66 | 65 | 28 |
| No. 11 | 91 | 91 | 44 |
| No. 12 | 129 | 135 | 139 |
| No. 13 | 141 | 147 | 95 |
| No. 14 | 157 | 214 | 164 |
| No. 15 | 172 | 169 | 115 |
| No. 16 | 176 | 187 | 159 |
| No. 17 | 195 | 259 | 161 |
| No. 18 | 205 | 394 | 276 |
| No. 19 | 233 | 250 | 240 |
| (Unit: IU/ml) | | | |

Considering that the measurement values for IgE concentration should be equivalent in samples that are not false-positive or false-negative, obvious false-positive results were seen with samples No. 1, 5, 9 and 18 with the reagent using the full-length antibody. On the other hand, the reagents using the CH3-deleted antibodies produced measurement values that generally matched the actual IgE concentration, and no obvious false positives were confirmed.

Industrial Applicability

With the present invention, it is possible to provide a reagent whereby non-specific reactions can be suppressed merely by deleting part of the Fc region in an antibody.

**Claims**

1. A reagent comprising an antibody from which part of CH3 or CH2 in the Fc region is deleted.

2. The reagent according to Claim 1, wherein all of CH3 or CH2 is deleted.

3. The reagent according to Claim 1, wherein all of CH2 is deleted and CH3 is bound directly or indirectly to a hinge region of the antibody.

4. The reagent according to any one of Claims 1 to 3, wherein the antibody is IgG.

5. The region according to any one of Claims 1 to 4, wherein a non-specific reaction of the antibody is suppressed due to the deletion of part of CH3 or CH2.

6. The reagent according to Claim 5, wherein the non-specific reaction is caused by a non-specific reaction factor that binds to the Fc region.

7. The reagent according to Claim 6, wherein the non-specific reaction factor is a complement C1q, Fcγ receptor or rheumatoid factor in a sample.

8. The reagent according to any of Claims 1 to 7, wherein the antigen-antibody reaction between the antibody and an antigen as a target for diagnosis is at least equivalent to that between the antigen and an antibody from which the Fc region is not deleted.

9. The reagent according to any of Claims 1 to 8, wherein the antibody is derived from a mouse, rabbit, rat, guinea pig, hamster, goat, sheep or camel.

10. The reagent according to any of Claims 1 to 9, wherein the antibody is a human antibody, humanized antibody or human chimera antibody.

11. The reagent according to any of Claims 1 to 9, wherein the antibody is a non-human chimera antibody.

12. The reagent according to any of Claims 1 to 11, wherein the reagent is for diagnosis.

13. The reagent according to any of Claims 1 to 11, wherein the reagent is for research.

14. A kit comprising the reagent according to any of Claims 1 to 11.

15. A kit comprising the reagent according to any of Claims 1 to 11 and a blocking agent.

16. The kit according to Claims 14 or 15, wherein the kit comprises a carrier.

17. The kit according to Claim 16, wherein the antibody is bound to a surface of the carrier.

18. The kit according to Claim 16 or 17, wherein the carrier is a microplate or latex.

19. The kit according to any of Claims 16 to 18, wherein the antibody is fixed on the carrier via a linker.

20. A method for suppressing non-specific reactions in antigen-antibody reactions, the method using an antibody from which part of CH3 or CH2 in the Fc region is deleted.

**Fig. 1**

**Fig. 2**

EP 4 130 032 A1

# Fig. 3

# Fig. 4

**Fig. 5**

## Fig. 6

Fig. 7

# Fig. 8

EP 4 130 032 A1

Fig. 9

EP 4 130 032 A1

**Fig. 10**

ΔCH3

R1
R2
R4
R10
R11
R13
R14
R15
R16
R17

Antibody addition

End of addition

Response (RU): 250, 200, 150, 100, 50, 0, -50

Time (s): 0, 40, 80, 120

## Fig. 11

# Fig. 12

**INTERNATIONAL SEARCH REPORT**

| International application No. |
| --- |
| PCT/JP2021/012724 |

A.  CLASSIFICATION OF SUBJECT MATTER

C07K  16/00(2006.01)i;  C07K  16/46(2006.01)i;  C12N  15/13(2006.01)i;  C12N
15/62(2006.01)i;  G01N  33/531(2006.01)i;  G01N  33/545(2006.01)i;  G01N
33/547(2006.01)i
FI:      G01N33/531 A ZNA; G01N33/545; G01N33/547; C12N15/13; C12N15/62 Z;
         C07K16/00; C07K16/46

According to International Patent Classification (IPC) or to both national classification and IPC

B.  FIELDS SEARCHED

Minimum documentation searched (classification system followed by classification symbols)

C07K16/00;  C07K16/46;  C12N15/13;  C12N15/62;  G01N33/531;  G01N33/545;
G01N33/547

Documentation searched other than minimum documentation to the extent that such documents are included in the fields searched
       Published examined utility model applications of Japan       1922–1996
       Published unexamined utility model applications of Japan     1971–2021
       Registered utility model specifications of Japan             1996–2021
       Published registered utility model applications of Japan     1994–2021

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | JP 11-514223 A (IMMUNOMEDICS, INC.) 07 December | 1-10, 12-17, |
|   | 1999 (1999-12-07) page 11, line 1 to page 57, line | 20 |
| Y | 16, fig. 1-8 | 19 |
| X | JP 2017-531426 A (MEDIMMUNE LIMITED) 26 October | 1-18, 20 |
| Y | 2017 (2017-10-26) paragraphs [0111]-[0224], | 19 |
|   | [0317]-[0407], fig. 1-10 | |
| X | US 2009/0155255 A1 (GLASER, S.) 18 June 2009 | 1-18, 20 |
|   | (2009-06-18) paragraphs [0169]-[0309], [0359]- | |
|   | [0420], fig. 1-36 | |

☒  Further documents are listed in the continuation of Box C.          ☒  See patent family annex.

| | |
| --- | --- |
| *   Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| 01 June 2021 (01.06.2021) | 22 June 2021 (22.06.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/012724 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | WO 2015/129651 A1 (FUJIREBIO INC.) 03 September 2015 (2015-09-03) paragraphs [0048]-[0056], fig. 1-7 | 1-3 |
| A | US 2011/0150894 A1 (INGBER, D.) 23 June 2011 (2011-06-23) entire text, all drawings | 1-20 |
| A | WO 2019/172401 A1 (NATIONAL INSTITUTE OF ADVANCED INDUSTRIAL SCIENCE AND TECHNOLOGY) 12 September 2019 (2019-09-12) entire text, all drawings | 1-20 |

Form PCT/ISA/210 (continuation of second sheet) (January 2015)

**INTERNATIONAL SEARCH REPORT**
Information on patent family members

| International application No. |
| --- |
| PCT/JP2021/012724 |

| Patent Documents referred in the Report | Publication Date | Patent Family | Publication Date |
| --- | --- | --- | --- |
| JP 11-514223 A | 07 Dec. 1999 | WO 1997/011370 A1 page 6, line 33 to page 57, line 6, fig. 1-8 EP 861440 A | |
| JP 2017-531426 A | 26 Oct. 2017 | WO 2016/050889 A1 paragraphs [0129]-[0234], [0311]-[0408], fig. 1-10 US 2017/0304343 A1 EP 3201233 A KR 10-2017-0062502 A CN 108064243 A | |
| US 2009/0155255 A1 | 18 Jun. 2009 | WO 2009/043051 A2 | |
| WO 2015/129651 A1 | 03 Sep. 2015 | TW 201536801 A JP 6512214 B2 | |
| US 2011/0150894 A1 | 23 Jun. 2011 | WO 2009/149239 A1 | |
| WO 2019/172401 A1 | 12 Sep. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S54119292 A

- JP 5015012 B

**Non-patent literature cited in the description**

- *Eur J Biochem.,* vol. 267 (24), 7246-57
- **XUEYONG ZHU et al.** A unique and conserved neutralization epitope in H5N1 influenza viruses identified by an antibody against the A/Goose/Guangdong/1/96 hemagglutinin. *Journal of virology,* 2013, vol. 87 (23), 12619-35

- **PENG H. et al.** Comparison of 5 monoclonal antibodies for immunopurification of human butyrylcholinesterase on Dynabeads: KD values, binding pairs, and amino acid sequences. *Chemico-Biological Interactions,* 2015, vol. 240, 336-345